# EUROPEAN PATENT APPLICATION

(11) **EP 4 564 253 A2**
(43) Date of publication of application: **04.06.2025**
(21) Application number: 25157810.0
(22) Date of filing: 28.07.2017
(51) Int. Cl.: G06Q 10/08

(54) **SYSTEM AND METHOD FOR INVENTORY SHARING IN A LABORATORY MANAGEMENT SYSTEM**

(30) Priority: 29.07.2016 US 201662368308 P
(62) Divisional of application: 17835321.5
(71) Applicant: Abbott Laboratories, Abbott Park, Illinois 60064-6008 (US)
(72) Inventor: BARNES, Kevin M., Gumee, 60031 (US); BIROLINI, Luca, 20090 Segrate (IT); WILLIAMS, William B., Coppell, 75019 (US); KHANAL, Deepak, Coppell, 75019 (US)
(74) Representative: Mathys & Squire

(57) **Abstract**

A system for sharing consumable inventory in a laboratory management system includes a middleware software component controlled by a processor, a plurality of instruments operatively coupled to the middleware software component by at least one communications network, and a consumable item configured to be removably installed in a first selected instrument of the plurality of instruments. The consumable item is used by the first selected instrument to perform tests specified by the laboratory management system where the first selected instrument partially depletes the consumable item. The first selected instrument is configured to update status and usage information regarding the consumable item. A consumables database is operatively coupled to the middleware software component and the processor is configured to store the updated status and usage information in the consumables database corresponding to the consumable item. Because the consumables database is accessible by the plurality of instruments, a second selected instrument of the plurality of instruments is able to perform tests using the consumable item, based on the corresponding updated status and usage information.

## Description

### 1. Cross-Reference To Related Applications.

This application claims the benefit of priority from provisional patent application Serial No. 62/368,308, filed on July 29, 2016, which is hereby incorporated by reference in its entirety.

### 2. Technical Field.

The present invention relates generally to a system and method for sharing reagent inventory among a plurality of instruments in a laboratory.

### 3. Background.

Laboratories use a variety of laboratory management systems to integrate laboratory software and instruments; to manage samples, laboratory users, and standards; to control other laboratory functions such as Quality Assurance (QA) and Quality Control (QC); to conduct sample planning, invoicing, and plate management; and to manage workflow. Laboratory management systems can include a variety of different types of systems for managing samples, information and/or instruments within a laboratory, such as a Laboratory Information System (LIS), a Process Development Execution System (PDES), and a Laboratory Information Management System or Laboratory Integration Management Solution (LIMS).

A Laboratory Information System ("LIS") is a class of software that receives, processes, and stores information generated by medical laboratory processes. LIS systems often must interface with instruments and other information systems such as hospital information systems (HIS). A Process Development Execution System (PDES) is a system which is used by companies to perform development activities for manufacturing processes.

A Laboratory Information Management System or Laboratory Integration Management Solution (LIMS) is a software system used in laboratories for the integration of laboratory software and instruments and the management of samples, laboratory users, standards and other laboratory functions such as Quality Assurance (QA) and Quality Control (QC), sample planning, invoicing, plate management, and workflow automation. LIMS systems may also support information gathering, decision making, calculation, review and release into the workplace and away from the office. More recently, LIMS systems are starting to expand into Electronic Laboratory Notebooks, assay data management, data mining and data analysis.

Laboratory instruments use a reagent to process and perform analysis on blood or tissue specimens. Reagents, also referred to as inventory, may be in the form of a container, a vial, a reagent pack, and the like. Reagents may require significant capital expenditure, and are subject to a shelf-life constraint (expiration date) and a limited-life constraint once opened for use, which may be significantly shorter than the shelf-life of an unopened reagent pack.

Each reagent or reagent pack is configured to provide reagent for a fixed number of tests or assays. Once a reagent pack is installed on an instrument, the instrument identifies the reagent pack and tracks and stores status information associated with that reagent pack. The instrument typically performs calibration and quality control tests for a newly installed reagent pack. Based on the status information, the instrument tracks total number of tests that the reagent pack is capable of providing, number of tests used, the number of tests remaining, the various expiration dates, and the like.

However, such information corresponds only to a particular reagent pack identified by a particular instrument on which that reagent pack has been installed. Accordingly, anytime a specific reagent pack is installed and then removed from an instrument, that instrument need only identify that specific reagent pack that was previously installed on that instrument, and then continue running further tests. In known systems, the specific reagent pack is run on one and only one instrument.

Specifically, if a reagent pack is removed from an instrument and later reinstalled in that same instrument, the instrument will identify the reagent pack, for example by reading a bar code label or an RFID (radio-frequency identification) tag, and will accurately track the remaining usage or number of tests remaining in the reagent pack.

However, the change in status of a reagent pack, such as number of tests used or remaining, shelf-life, and the like, are not saved or stored back to the reagent pack packaging or to a central location. The reagent pack itself has no active memory. Such information is stored only locally in the particular instrument that is associated with the specific reagent pack. There currently exists a one-to-one relationship between a particular instrument and the corresponding reagent pack. Thus, if a partially used reagent pack is installed on a different instrument, the new instrument will not be able to recognize that the reagent pack has been partially used, and will treat the reagent pack as a new reagent pack, even though it had been partially depleted. This will cause errors when the reagent pack becomes depleted after apparently supplying fewer than the expected number of tests.

Known instruments and laboratory information systems do not share the reagent pack history from instrument to instrument or with a central database. Rather, such reagent pack history is local to one instrument, based on the bar code identifier (or other identifier) corresponding to the reagent pack.

Accordingly, it is desirable to provide an instrument with accessible status and tracking information regarding a reagent pack when the reagent pack is used on multiple instruments. The ability for instruments to "share" reagent packs would increase the efficiency and flexibility of the instrument and the laboratory environment because a particular test using a particular reagent may be performed only infrequently. Removal of such a reagent pack from a first instrument for future use on a second instrument would allow the first instrument to perform other tests using different reagent packs. In one embodiment, if the infrequently-performed test is to be performed again, the partially-used reagent pack may be retrieved and used again, perhaps in a different instrument.

### SUMMARY

In one embodiment, a system for sharing consumable inventory in a laboratory management system includes a middleware software component controlled by a processor, a plurality of instruments operatively coupled to the middleware software component by at least one communications network, and a consumable item configured to be removably installed in a first selected instrument of the plurality of instruments. The consumable item may be used by the first selected instrument to perform tests specified by the laboratory management system, where the first selected instrument partially depletes the consumable item. The first selected instrument may be configured to update status and usage information regarding the consumable item. A consumables database is operatively coupled to the middleware software component and the processor is configured to store the updated status and usage information in the consumables database corresponding to the consumable item. Because the consumables database is accessible by the plurality of instruments, a second selected instrument of the plurality of instruments is able to perform tests using the consumable item, based on the corresponding updated status and usage information retrieved from the consumables database.

The ability to share reagent inventory among multiple instruments in a laboratory increases efficiency and reduces costs. In known systems, a reagent or reagent pack must be used on one and only one instrument. This is in part due to the limitations of known, commercially-available, industry standard laboratory software systems. In such known systems, if a low volume test is performed and no further tests of that kind are called for before the expiration date of the reagent pack, the remaining reagent goes unused and expires. This can be very costly and inefficient. In accordance with one embodiment, because the reagent may be shared among several instruments, a test of a particular kind, even if low volume, may be run on different instruments. Further, if one instrument becomes disabled while performing one type of test, the reagent pack may be removed from the disabled instrument and installed on another similar or identical instrument so that the testing can continue. Inventory sharing enables sharing of reagents, calibration, and control material across instrument modules while preserving usage and stability data.

The scope of the present invention is defined solely by the appended claims and is not affected by the statements within this summary.

### BRIEF DESCRIPTION OF THE DRAWINGS

The invention can be better understood with reference to the following drawings and description. The components in the figures are not necessarily to scale, emphasis instead being placed upon illustrating the principles of the invention.
FIG. 1 is a block diagram of an exemplary computing system, in accordance with one embodiment of the present invention.
FIG. 2 is an illustration of a laboratory with instruments connected with a computer running a laboratory management system, in accordance with one embodiment.
FIG. 3 is a block diagram showing a reagent-sharing module coupled to an LIS, in accordance with one embodiment.
FIGS. 4-5 show configuration block diagrams, in accordance with one embodiment.
FIG. 6 depicts an overview of the inventory sharing arrangement, in accordance with one embodiment.

### DETAILED DESCRIPTION

The present invention may generate an instrument information representation in response to receiving information generated by an instrument and display the instrument information representation on a display. Users of laboratory management systems may visually and quickly check the status of an instrument and may see information presented in a graphical manner which details the QC results of a particular instrument.

In the description that follows, the subject matter of the application will be described with reference to acts and symbolic representations of operations that are performed by one or more computers, unless indicated otherwise. As such, it will be understood that such acts and operations, which are at times referred to as being computer-executed, include the manipulation by the processing unit of the computer of electrical signals representing data in a structured form. This manipulation transforms the data or maintains it at locations in the memory system of the computer which reconfigures or otherwise alters the operation of the computer in a manner well understood by those skilled in the art. The data structures where data is maintained are physical locations of the memory that have particular properties defined by the format of the data. However, although the subject matter of the application is being described in the foregoing context, it is not meant to be limiting as those skilled in the art will appreciate that some of the acts and operations described hereinafter can also be implemented in hardware, software, and/or firmware and/or some combination thereof.

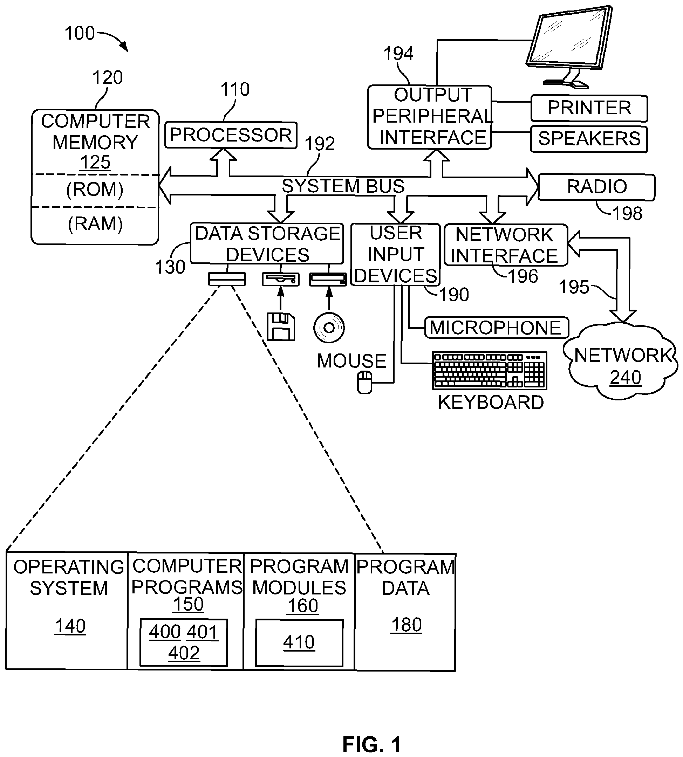

With reference to FIG. 1, depicted is an exemplary computing system for implementing embodiments. FIG. 1 includes computer 100 running a computer program 150, such as a laboratory management system (LMS) software application 400. The LMS software application 400 includes software applications such as a Laboratory Information Management System (LIMS) software application 401, a Laboratory Information System (LIS) software application 402, or a Process Development Execution System (PDES) software application and the like.

The LIMS software application 401 is a software application used in laboratories for the integration of laboratory software and instruments and the management of samples, laboratory users, standards and other laboratory functions such as Quality Assurance (QA) and Quality Control (QC), sample planning, invoicing, plate management, and workflow automation. The LIS software application 402 is a software application that receives, processes, and stores information generated by medical laboratory processes. The LIS software application 402 often must interface with instruments and other information systems such as hospital information systems (HIS). The LIS software application 402 is a highly configurable application which is customized to facilitate a wide variety of laboratory workflow models. The PDES software application 403 is a software application which is used by companies to perform development activities for manufacturing processes.

The computer 100 includes a processor 110 in communication with a computer readable memory medium 120. Computer readable memory medium 120 is any medium which can be used to store information which can later be accessed by processor 110. Computer readable memory medium 120 includes computer memory 125 and data storage devices 130. Computer memory 120 is preferably a fast-access memory and is used to run program instructions executable by the processor 110. Computer memory 120 includes random access memory (RAM), flash memory, and read only memory (ROM). Data storage devices 130 are preferably physical devices and are used to store any information or computer program which may be accessed by the processor 110, such as an operating system 140, computer programs 150 such as LMS software application 400, program modules 160 such as a graphical display module 410 which runs as a part of LMS software application 400, and program data 180. Data storage devices 130 and their associated computer readable memory medium provide storage of computer readable instructions, data structures, program modules and other data for the computer 100. Data storage devices 130 include magnetic medium like a floppy disk, a hard disk drive, and magnetic tape; an optical medium like a Compact Disc (CD), a Digital Video Disk (DVD), and a Blu-ray Disc; and solid state memory such as random access memory (RAM), flash memory, and read only memory (ROM).

Computer 100 further includes input devices 190 through which data may enter the computer 100, either automatically or by a user who enters commands and data. Input devices 190 can include an electronic digitizer, a flatbed scanner, a barcode reader, a microphone, a camera, a video camera, a keyboard and a pointing device, commonly referred to as a mouse, a trackball or a touch pad, a pinpad, any USB device, any Bluetooth enabled device, an RFID or NFC device, and a debit card reader. Other input devices may include a joystick, game pad, satellite dish, scanner, an instrument, a sensor, and the like. In one or more embodiments, input devices 190 are portable devices that can direct display or instantiation of applications running on processor 110.

These and other input devices 190 can be connected to processor 110 through a user input interface that is coupled to a system bus 192, but may be connected by other interface and bus structures, such as a parallel port, game port or a universal serial bus (USB). Computers such as computer 100 may also include other peripheral output devices such as speakers, printers, and/or display devices, which may be connected through an output peripheral interface 194 and the like.

Computer 100 also includes a radio 198 or other type of communications device for wirelessly transmitting and receiving data for the computer 100 with the aid of an antenna. Radio 198 may wirelessly transmit and receive data using WiMAXTM, 802.11a/b/g/n, BluetoothTM, 2G, 2.5G, 3G, and 4G, wireless standards.

Computer 100 may operate in a networked environment 195 using logical connections to one or more remote computers, such as a remote server 240. The remote server 240 may be a personal computer, a server, a router, a network PC, a peer device or other common network node, and may include many if not all of the elements described above relative to computer 100. Networking environments are commonplace in offices, enterprise-wide computer networks, intranets and the Internet. For example, in the subject matter of the present application, computer 100 may comprise the source machine from which data is being migrated, and the remote computer may comprise the destination machine. Note, however, that source and destination machines need not be connected by a network or any other means, but instead, data may be migrated via any media capable of being written by the source platform and read by the destination platform or platforms. When used in a LAN or WLAN networking environment, computer 100 is connected to the LAN or WLAN through a network interface 196 or an adapter. When used in a WAN networking environment, computer 100 may include a modem or other means for establishing communications over the WAN, such as radio 198, to environments such as the Internet or to another remote computer. It will be appreciated that other means of establishing a communications link between computer 100 and other remote computers may be used.

In one embodiment, computer 100 is in communication with remote server 240, and the LMS software application 400 is run on the remote server 240, receiving commands and information from the computer 100 being input by a user. Information from the LMS software application 400 running on the remote server 240 is displayed on a display connected with the computer 100.

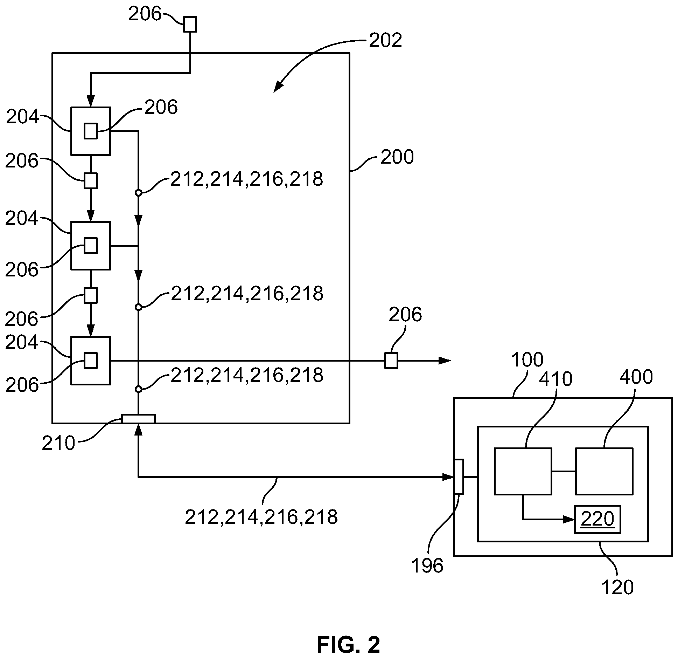

With reference to FIGS. 1-2, a graphical display module 410 is provided for graphically generating an instrument information representation 220 in response to receiving information 212 generated by one or more instruments 204. The graphical display module 410 is connected with or runs within a laboratory management system (LMS) software application 400 used to manage a laboratory 200. Laboratory 200 is any place of manufacture or place of analyses where actions or tests are performed on samples 206 using equipment or instruments 204. Laboratory 200 includes various types of laboratories such as medical or clinical laboratories, biological laboratories, chemistry laboratories, chemical or petroleum laboratories, commercial or manufacturing plants, forensics or crime laboratories, pathology laboratories, public safety and public health laboratories, and water processing and testing facilities. Samples 202 are any object which enters a laboratory 200 upon which an action or test is performed. Samples 202 include: biological samples taken from a patient, such as blood, urine or tissue; evidence samples taken from a crime scene, such as bullets, biological samples, pictures, and video; samples of materials, liquids, or compounds; and parts or components. Instruments 204 are any type of equipment which can perform an action or an analyses or test on a sample 206, and include laboratory instruments, manufacturing equipment such as welding tools and robotic arms, sensors such as temperature sensors and weight sensors, and imaging equipment such as bar code scanners or cameras.

As the instruments become operational and as samples 206 are routed in and out of the laboratory 200, information 212 may be generated by the instrument 204 and transmitted to the LMS software application 400. Information 212 may include: status information 214 which details the status of an instrument 204 including any error messages received from an instrument 204 and any information as to the current operating state of an instrument 204, instrument information which includes information describing the instrument such as the type and model number of the instrument 204; the current workload of an instrument 204 which includes how many jobs an instrument 204 may have in its queue; quality control (QC) information 216 generated by the instrument 204 for QC samples, and results information 218. An instrument information representation 220, as discussed herein, may display real time QC status of each assay performed on an instrument 204. If any assay fails any QC rules defined in the LMS software application 400 using, for example, Westguard Rules, Custom Rules or Moving Average Rules will be applied to QC information 216 and displayed by the instrument information representation 220 so that a user will be alerted as to a failure of the instrument 204 and the user will be able to view in real time the QC information 216 on a display.

As samples 206 are routed to a particular instrument 204 within the laboratory 200, tests or actions are performed on the sample 206, and results information 218 associated with the sample 206 may be generated by the instrument 204 and transmitted to the LMS software application 400. Results information 218 is generated by an instrument 204 in the laboratory 200 and is associated with or is from performing a test or action on the sample 206, and includes things as test results or sample properties, and any other information 208 which may be associated with the sample 206 and obtained from the sample 206 by the instrument 204. Results information 218 is eventually entered into a database managed by the LMS software application 400.

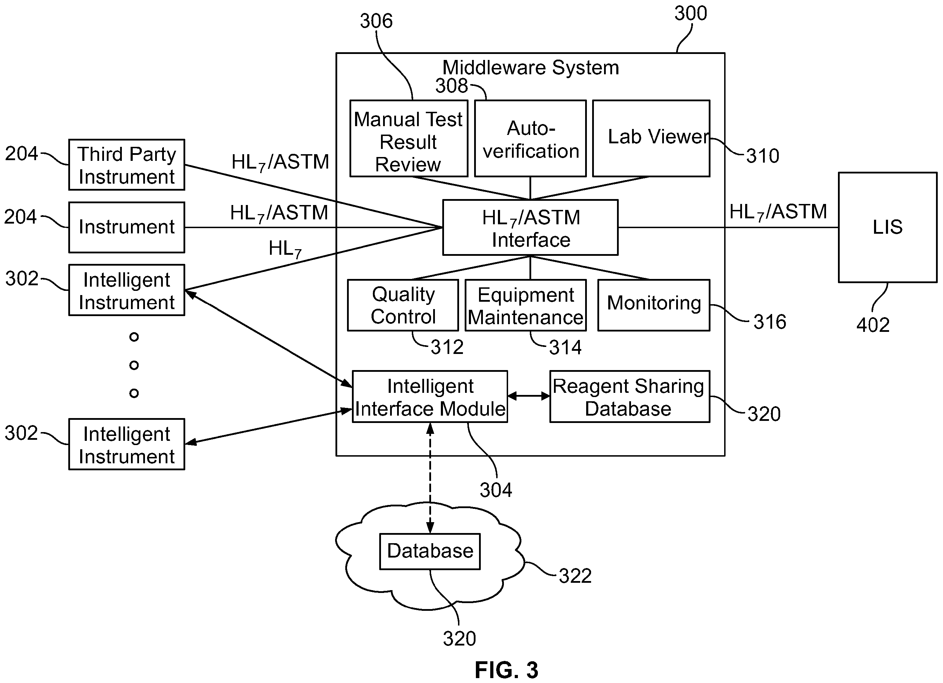

FIG. 3 is a block diagram showing a middleware system 300 that may be operatively coupled to an LIS 402. Alternatively, the middleware system 300 may be incorporated into the LIS 402. Once such middleware system or application 300 may be the AMS platform (also referred to as the AlinIQ AMS system) available from Abbott Laboratories. The middleware system 300 may be coupled to the laboratory instruments 204 by various communication protocols, such as HL7 (Health Level 7) and ASTM (American Society On The International Associate For Testing and Materials). Such protocols are industry-standard protocols, and most if not all laboratory instruments 204 may communicate using these protocols. The middleware system 300 may include an HL7 or ASTM interface adapted to communicate with instruments having HL7 or ASTM capability. The middleware system 300 may be a software component that interfaces with and communicates with instruments so as to send and receive data, commands, status, and the like.

The instruments may also include instruments having a non-industry or proprietary standard interface, such as one or more of intelligent instruments 302 shown in Fig. 3, which may be operatively coupled to an intelligent interface module 304 of the middleware system 300. The intelligent interface module 304 may be part of the middleware system 300, incorporated into the middleware system 300, or remotely and operatively coupled to the middleware system 300. The intelligent instruments 302 may also include the known industry-standard interfaces as well, such as HL7 and ASTM described above.

The middleware system 300 may include a manual test results viewer 306, an auto-verification module 308, a lab viewer 310, a quality control module 312, an equipment maintenance module 314, and a monitoring module 316. The lab viewer 310 may be used to view specific operation and results of the various connected instruments 204, 302.

The intelligent instruments 302 coupled to the intelligent interface module 304 provide the ability to share reagents or inventory among and between the various intelligent instruments 302. As described above, such reagent sharing is not possible using the standard instruments 204 having the standard HL7 and ASTM interfaces. The intelligent interface module 304 is configured to provide the robust communications protocol to facilitate reagent or inventory sharing between the intelligent instruments 302.

The intelligent interface module 304 is operatively coupled to and is able to access a reagent-sharing database or memory 320 (consumables database), which may be local to the intelligent interface module 304 or which may be remote from the intelligent interface module 304. Thus, the reagent-sharing database or memory 320 may be a cloud-based 322 remote database. Reagent sharing permits laboratory staff to seamlessly share partially used inventory (reagent packs) between intelligent instruments 302 or work cells so as to save time and costs required to maintain and track separate inventory stock for each instrument

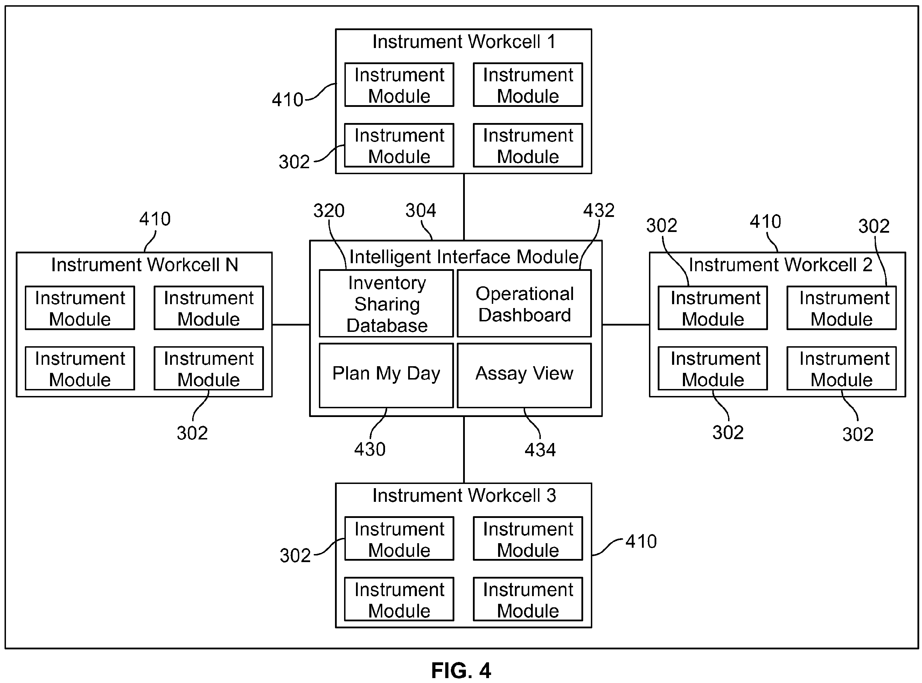

As shown in Figs. 3 and 4, the intelligent interface module 304 may be operatively coupled to, or include the reagent-sharing database 320. Further, Fig. 4 shows a plurality of workcells 410 operatively coupled to the intelligent interface module 304. Any suitable number of workcells 410 may be coupled to the intelligent interface module 304 depending on the application and testing environment. Each workcell 410 may be a cluster of intelligent instruments 302. Workcells 410 typically include from one to four intelligent instruments 302, but other configurations are possible. Intelligent instruments 302 can be clustered together as the application permits, but returns on efficiency may not be realized for clusters greater than four instruments.

Intelligent instruments 302 may be clustered together in a workcell 410 to increase efficiency and throughput, and each workcell may include intelligent instruments 302 of the same or identical type, or may include different kinds or models of intelligent instruments 302. The intelligent interface module 304 of Fig. 4 illustrates the flexibility of the intelligent interface module 304, and in some embodiments, may include other software applications, such as a daily planner referred to in the figure as Plan My Day 430, an Operational Dashboard 432, and an Assay Viewer 434.

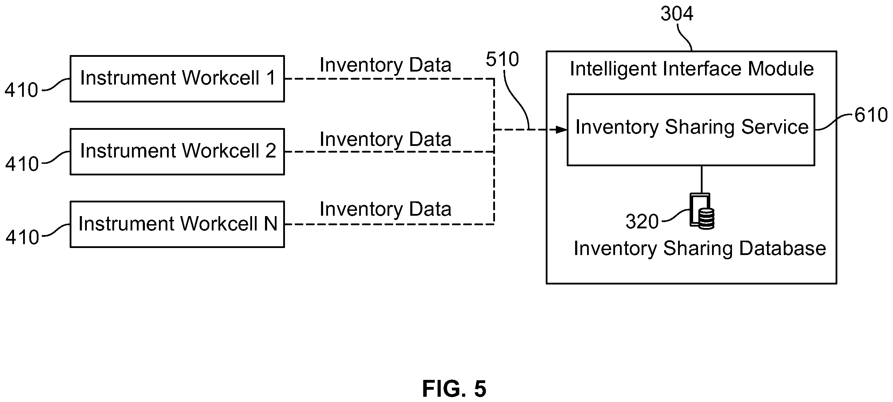

Fig. 5 shows certain data flow associated with the intelligent interface module 304. One or more workcells 410 operatively coupled to the intelligent interface module 304 communicate inventory data 510 to the intelligent interface module 304. Such information is stored and updated in the inventory or reagent sharing database 320. Such communication is handled via a proprietary protocol because the industry-standard communication protocols, such as HL7 and ASTM, are not sufficiently robust to provide, receive, or track, such reagent information. Existing known protocols, such as HL7 and ASTM cannot support reagent sharing information.

As described above, the intelligent interface module may communicate with the intelligent instruments 302 via a custom communication protocol capable of transmitting reagent information, which is different than the industry-standard protocol used by the HL-7 or ASTM network. The custom protocol, may not be compatible with the HL-7 and the ASTM protocol, and is much more robust and flexible than the HL-7 and ASTM protocol. The HL-7 and ASTM protocols are limited in nature because they are intended to be general purpose, and are compatible with virtually any and all instruments 204 that are connected to an LIS. Because the HL-7 and ASTM protocols are designed to be widely compatible, they are inherently inflexible and support a very limited data set.

For example, the HL-7 and ASTM protocol in existing instrument-coupled-to-middleware systems do not support reporting of reagent status and usage information, which information dynamically changes during the instrument processing. The status and reagent usage information may include, among other information: 1) an identity of the consumable item, 2) number of total tests corresponding to the consumable item, 3) the number of tests remaining in the consumable item, 4) shelf-life expiration of the consumable item, 5) remaining lifetime of the consumable item since initial opening of the consumable item, 6) quality control information relating to the consumable item, 7) calibration information relating to the consumable item, and the like.

Typically, the identity of the consumable item or reagent container may be obtained via a bar-code label attached to the reagent container, which is scanned by the intelligent instrument when loaded. Alternatively, the reagent container may include an RFID tag that provides the identity of the consumable item.

Each consumable item may inherently include a fixed number of tests that it can support before being depleted. The intelligent interface module 304 in communication with each intelligent instrument 302 tracks all such related information. For example, when a consumable item is initially loaded on an intelligent instrument 302 and its identity is initially established, the maximum number of tests before depletion is obtained from that consumable item, and updated in the consumables database 320. As various tests are conducted using that consumable item, the number of test conducted (corresponding to an amount of reagent used) is tracked, and the consumables database 320 is updated. In one example, the consumable item may be used until depleted, which would then cause the testing to be interrupted pending notification that the consumable item is depleted. In another example, testing using that consumable item is either interrupted or completed, but the consumable item is not yet depleted and may be used at a later time. Accordingly, the number of tests used corresponding to the identity of that consumable item is recorded so that that consumable item may be used in subsequent testing, perhaps on a different intelligent instrument 302. Thus, the total number of tests or maximum number corresponding to the consumable item is recorded, as well as the number of tests remaining.

Lifetime information is also saved in the consumables database 320 corresponding to each identified consumable item. Such information includes maximum shelf-life of the reagent, for example, 24 months, and the shelf-life once opened, which will be some value less than the maximum shelf-life. For example, if a consumable item has been previously used on a first instrument, and subsequently stored, and then reinstalled on a second instrument at a later time, the lifetime information is retrieved from the consumables database 320 to verify that the shelf-life once opened value has not been exceeded. If exceeded, the consumable item is deemed stale and its use will not be permitted to be used.

Regarding lifetime values for date/time, the format of the values may be JSON-type and may be IS0-8601 compliant. The date and time values are created when the item is created and is fixed and is based on the lot expiration dates of the specific reagent.

The above-described status and reagent usage information in known systems cannot be stored or retrieved external to the instrument performing the test because of the limitations of the existing protocols, like HL-7 and ASTM, do not and cannot support such information transfer. However, the custom protocol used by the intelligent interface module 304 is designed to support the transfer of such reagent usage and status information, thus such information is stored in a central repository, such as the reagent-sharing or consumables database 320. Because the reagent sharing database 320 is accessible to the intelligent interface module 304, such data may be retrieved from or supplied to any intelligent instrument 302 to which the middleware system 300 is coupled. This permits a reagent pack to be shared or moved between such intelligent instruments 302.

For example, a consumable item, such as a reagent pack, may be removably installed in a first selected intelligent instrument 302 of a plurality of intelligent instruments, which may be located in the same or in a different workcell 410. The reagent pack may be used by a first selected intelligent instrument 302 to perform tests specified by the LIS. A selected intelligent instrument 302 may update status and usage information regarding the reagent pack. This may occur in real-time or in near real-time. Periodic updates may occur frequently, such as several times per second. As the intelligent instrument 302 conducts the specified tests, the intelligent interface module 304 may retrieve and store the updated status and usage information in the reagent-sharing database 320 corresponding to the reagent pack.

For various reasons it may be desirable to remove a reagent pack from a particular intelligent instrument 302, whether or not the reagent pack has been completely depleted or not. In one example, all requested tests using a particular reagent pack may have been completed, even though the reagent pack was only partially depleted. Alternatively, an instrument failure may require that the intelligent instrument 302 currently in operation go off-line. Because the reagent-sharing database 320 is accessible by any and all of the intelligent instruments 302 via the intelligent interface module 304, the partially depleted reagent pack described in the example above may be removed from the first intelligent instrument 302 and installed on a second selected intelligent instrument 302 if continued tests are to be performed using the removed reagent pack.

The updated status and usage information regarding the partially depleted reagent pack may be retrieved from the reagent-sharing database 320 and provided to the second intelligent selected instrument 302 in which the partially depleted reagent pack is installed. The second selected intelligent instrument 302 may then continue to perform tests using the partially depleted reagent pack removed from the first intelligent instrument 302. In this way, reagent packs are used in the most efficient and economical way possible, with as little waste as possible.

In some embodiments, when a partially depleted reagent pack is removed from a first intelligent instrument 302 and installed on a second intelligent instrument 302, the second intelligent instrument 302 may perform QC and calibration corresponding to the partially depleted reagent pack. This is particularly true if the partially depleted reagent pack has never been previously installed on that second intelligent instrument 302. The reagent-sharing database 302 may store QC, calibration, and status information for each intelligent instrument 302 in which a particular reagent pack has been installed.

Inventory or reagent sharing allows inventory items to be shared across multiple instruments. The intelligent interface module 304 facilitates portability by centrally tracking and updating the state information about consumed inventory items in the reagent-sharing database 320 that each intelligent instrument 302 accesses to obtain the latest state on an inventory item.

Reagent inventory sharing involves an inventory item, such as a reagent pack and calibration/control material, which may be loaded onto an intelligent instrument 302 in a workcell 410. Ownership of the item is registered to the workcell 410 in the reagent-sharing database 320. When the item is unloaded, the item information in the intelligent interface module 304 may be updated with the usage information (e.g. test count, etc.). When the item is loaded into another workcell 410, the workcell obtains the latest usage/stability information from intelligent interface module 304 before proceeding with the consumption of the item. This also provides a mechanism for asynchronous updates and reconciliation of inventory, in case of temporary errors or offline status. Note that the same instance of intelligent interface module 304 can support multiple types of instruments in a lab.

The reagent-sharing database 320 may be a passive database with no awareness of the type or instance of the connected intelligent instruments 302. Preferably, it is the responsibility of the workcell 410 to ensure a unique identity of each inventory item, so that the sharing is limited to 'like' types.

Referring to Figs. 4-6, the communications format between the workcell 410 and the intelligent interface module 304 may use an HTTP-based scheme known as Representational State Transfer (REST). A server may exposes secure hypertext transfer protocol (HTTPS) endpoints to facilitate inventory sharing services. The transmitted payload may be represented in the form of Javascript Object Notation (JSON) data. The workcell hosts components that are specifically designed to broker communication with their remote service endpoints. These components are used by other components as needed to fulfil the business requirements.

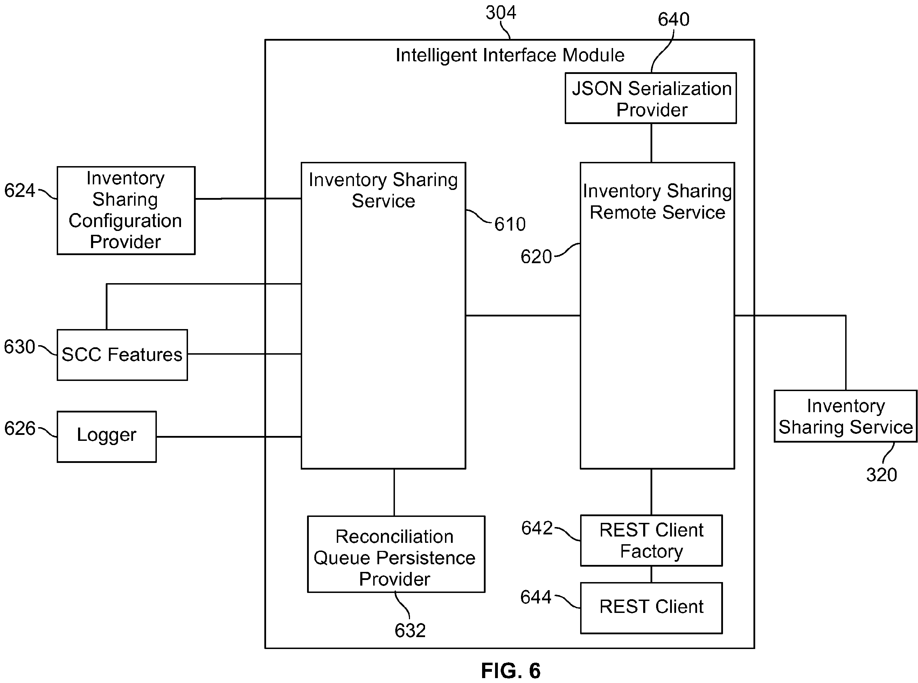

Referring to Fig. 6, another embodiment of the intelligent interface module in an inventory configuration is shown. The intelligent interface module 304 may include an inventory sharing service 610 and a remote inventory sharing service 620. The inventory sharing service 610 may be operatively coupled to an inventory sharing configuration provider 624, a data logger 626, a service control center (SCC) features module 630, and a reconciliation queue provider 632. The remote inventory sharing service 620 may be coupled to a JSON serialization interface 640, a REST client factory interface 642, and a REST client module 644

The remote inventory sharing service 620 allows a seamless interface between the remote service portions of the intelligent interface module 304 and remote sharing. The service control center (SCC) features module 630 is essentially the computer responsible for the data processing in a particular workcell 410.

The reconciliation queue provider 632 provides queue management for sharing requests. The inventory sharing service 610 persists requests for inventory information reconciliation in a queue, where each item in the queue represents an item that needs to be reconciled, and this queue is persistent. Rather than assume a particular persistence technology, embodiments described herein permit the consuming application to fulfill this as an interface dependency.

The JSON serialization interface 640 provides the serialization/deserialization functionality to transform inventory information to/from Javascript Object Notation (JSON) representation for use in the communications protocol. The REST client factory interface 642 handles communication to and from the workcell 410 using a representational state transfer (REST) scheme over secure Hypertext Transfer Protocol (HTTPS). Inventory sharing components described herein may rely on specialized REST client operations to establish communication. The REST client factory interface 642 builds and supplies the REST client objects to the inventory sharing service 610.

The term "reagent" or "reagent sharing" in some embodiments is not necessarily limited to an actual reagent chemical, but may be more general in nature, and may encompass any consumable item used in a laboratory instruments, such as reagents, calibrators, controls or other consumable inventory item.

In one embodiment, the intelligent interface module 304 may be integrated with the LIMS. In another embodiment, the intelligent interface module 304 may be separate from the LIMS. In another embodiment, the intelligent interface module 304 is located within a hospital or laboratory computer system. In another embodiment, the intelligent interface module 304 may be distributed over multiple instrument computer control systems. In this way, a particular intelligent instrument 302 having a database that tracks reagent information used on that intelligent instrument may broadcast updates to that instrument's database to other instruments on the same communication network. Each instrument subscribing to the broadcast updates may update its database tracking reagent information. Thus, reagent usage information is "shared" among different instruments.

Further, in some embodiments, a centralized reagent-sharing database may not be required. To replace the central reagent-sharing database 320, a publish/subscribe technique may be used to share reagent or other inventory information among a plurality of instruments. In such a specific embodiment, each intelligent instrument 302 may locally store the reagent information for all inventory items. As the status of each inventory item changes, the corresponding intelligent instrument 302 broadcasts that status change on the network. Other intelligent instruments 302 would be configured to listen for those messages and would receive and process each message when broadcast, and update their local corresponding reagent-sharing database accordingly. In that way every intelligent instrument 302 would store and retain the reagent/status information for all other instruments.

In other embodiment, a point-to-point method may be used to obviate the need for a centralized consumables database 320. In such an embodiment each intelligent instrument 302 may be configured with the IP address of all other relevant intelligent instruments 302 on the network, and message updates/status information may sent by each intelligent instrument 302 directly to all the other instruments defined in that list.

Those having skill in the art will recognize that the state of the art has progressed to the point where there is little distinction left between hardware and software implementations of aspects of systems; the use of hardware or software is generally (but not always, in that in certain contexts the choice between hardware and software can become significant) a design choice representing cost vs. efficiency tradeoffs. Those having skill in the art will appreciate that there are various vehicles by which processes and/or systems and/or other technologies described herein can be effected (e.g., hardware, software, and/or firmware), and that the preferred vehicle will vary with the context in which the processes and/or systems and/or other technologies are deployed. For example, if an implementer determines that speed and accuracy are paramount, the implementer may opt for a mainly hardware and/or firmware vehicle; alternatively, if flexibility is paramount, the implementer may opt for a mainly software implementation; or, yet again alternatively, the implementer may opt for some combination of hardware, software, and/or firmware. Hence, there are several possible vehicles by which the processes and/or devices and/or other technologies described herein may be effected, none of which is inherently superior to the other in that any vehicle to be utilized is a choice dependent upon the context in which the vehicle will be deployed and the specific concerns (e.g., speed, flexibility, or predictability) of the implementer, any of which may vary. Those skilled in the art will recognize that optical aspects of implementations will typically employ optically-oriented hardware, software, and or firmware.

The foregoing detailed description has set forth various embodiments of the devices and/or processes via the use of block diagrams, flowcharts, and/or examples. Insofar as such block diagrams, flowcharts, and/or examples contain one or more functions and/or operations, it will be understood by those within the art that each function and/or operation within such block diagrams, flowcharts, or examples can be implemented, individually and/or collectively, by a wide range of hardware, software, firmware, or virtually any combination thereof. In one embodiment, several portions of the subject matter described herein may be implemented via Application Specific Integrated Circuits (ASICs), Field Programmable Gate Arrays (FPGAs), digital signal processors (DSPs), or other integrated formats.

However, those skilled in the art will recognize that some aspects of the embodiments disclosed herein, in whole or in part, can be equivalently implemented in integrated circuits, as one or more computer programs running on one or more computers (e.g., as one or more programs running on one or more computer systems), as one or more programs running on one or more processors (e.g., as one or more programs running on one or more microprocessors), as firmware, or as virtually any combination thereof, and that designing the circuitry and/or writing the code for the software and or firmware would be well within the skill of one of skill in the art in light of this disclosure. In addition, those skilled in the art will appreciate that the mechanisms of the subject matter described herein are capable of being distributed as a program product in a variety of forms, and that an illustrative embodiment of the subject matter described herein applies regardless of the particular type of signal bearing medium used to actually carry out the distribution. Examples of a signal bearing medium include, but are not limited to, the following: a computer readable memory medium such as a magnetic medium like a floppy disk, a hard disk drive, and magnetic tape; an optical medium like a Compact Disc (CD), a Digital Video Disk (DVD), and a Blu-ray Disc; computer memory like random access memory (RAM), flash memory, and read only memory (ROM); and a transmission type medium such as a digital and/or an analog communication medium like a fiber optic cable, a waveguide, a wired communications link, and a wireless communication link.

The herein described subject matter sometimes illustrates different components contained within, or connected with, different other components. It is to be understood that such depicted architectures are merely exemplary, and that in fact many other architectures can be implemented which achieve the same functionality. In a conceptual sense, any arrangement of components to achieve the same functionality is effectively "associated" such that the desired functionality is achieved. Hence, any two components herein combined to achieve a particular functionality can be seen as "associated with" or "connected with" each other such that the desired functionality is achieved, irrespective of architectures or intermediate components. Likewise, any two components so associated can also be viewed as being "operably connected", or "operably coupled", to each other to achieve the desired functionality, and any two components capable of being so associated can also be viewed as being "operably couplable", to each other to achieve the desired functionality. Specific examples of operably couplable include but are not limited to physically mateable and/or physically interacting components and/or wirelessly interactable and/or wirelessly interacting components and/or logically interacting and/or logically interactable components.

While particular aspects of the present subject matter described herein have been shown and described, it will be apparent to those skilled in the art that, based upon the teachings herein, changes and modifications may be made without departing from the subject matter described herein and its broader aspects and, therefore, the appended claims are to encompass within their scope all such changes and modifications as are within the true spirit and scope of the subject matter described herein. Furthermore, it is to be understood that the invention is defined by the appended claims. Accordingly, the invention is not to be restricted except in light of the appended claims and their equivalents.

The present invention is further defined by the following clauses:
1. A system for sharing consumable items in a laboratory management system, the system for sharing comprising:
   a middleware software component controlled by a processor;
   a plurality of instruments operatively coupled to the middleware software component by at least one communications network;
   a consumables database operatively coupled to the middleware software component;
   the consumable item configured to be removably installed in a first selected instrument of the plurality of instruments, the consumable item used by the first selected instrument to perform tests specified by the laboratory management system, the first selected instrument partially depleting the consumable item;
   the first selected instrument configured to update status and usage information regarding the consumable item;
   the processor configured to store the updated status and usage information in the consumables database corresponding to the consumable item; and
   wherein the consumables database is accessible by the plurality of instruments such that a second selected instrument of the plurality of instruments performs tests using the partially depleted consumable item, based on the corresponding updated status and usage information.
2. The system of clause 1, wherein the consumable item is a reagent kit or reagent pack.
3. The system of clause 1, wherein the consumables database is part of the middleware software component.
4. The system of clause 1, wherein consumables database is a cloud-based database separate and apart from the middleware software component.
5. The system of clause 1, wherein the status and usage information includes an identity of the consumable item, number of total tests corresponding to the consumable item, the number of tests remaining in the consumable item, shelf-life expiration of the consumable item, remaining lifetime of the consumable item since initial opening of the consumable item, quality control information relating to the consumable item, and calibration information relating to the consumable item.
6.The system of clause 1, wherein portions of the status and usage information change dynamically over time as the selected instrument conducts tests.
7. The system of clause 1, wherein the plurality of instruments are operatively coupled to the middleware software component by an HL7 or an ASTM communication network.
8. The system of clause 7, wherein the HL7 and ASTM communication networks permit communication between the middleware software component and legacy instruments.
9. The system of clause 1, wherein at least one instrument of the plurality of instruments is operatively coupled to the middleware software component by a communication network using a custom network protocol.
10. The system of clause 9, wherein the processor stores the status and usage information regarding the consumable item in the consumables database via the communication network using the custom network protocol.
11. The system of clause 1, wherein the partially depleted consumable item is removed from a first instrument and installed on a second instrument, and wherein the second instrument obtains corresponding status and usage information regarding the partially depleted consumable item from the consumables database, and performs further tests using the partially used consumable item.
12. A method for sharing reagent inventory in a laboratory management system, the method comprising:
   providing a middleware software component controlled by a processor;
   operatively coupling a plurality of instruments to the middleware software component via at least one communications network;
   removably installing a consumable item in a first selected instrument of the plurality of instruments, the consumable item used by the first selected instrument to perform tests specified by the laboratory management system, the first selected instrument partially depleting the consumable item;
   updating, by the first selected instrument, status and usage information regarding the consumable item;
   operatively coupling a consumables database to the middleware software component;
   storing, by the processor, the updated status and usage information in the consumables database corresponding to the consumable item; and
   wherein the consumables database is accessible by the plurality of instruments such that a second selected instrument of the plurality of instruments is able to perform tests using the partially depleted consumable item, based on the corresponding updated status and usage information stored in the consumables database.
13. A non-transitory computer readable memory medium comprising program instructions for sharing reagent inventory in a laboratory management system, the program instructions executed by a processor to:
   provide a middleware software component;
   operatively couple a plurality of instruments to the middleware software component via at least one communications network;
   updating, by a first selected instrument, status and usage information regarding a consumable item removably installed in the first selected instrument of the plurality of instruments, the consumable item used by the first selected instrument to perform tests specified by the laboratory management system, the first selected instrument partially depleting the consumable item;
   operatively coupling a consumables database to the middleware software component; and
   storing the updated status and usage information in the consumables database corresponding to the consumable item, wherein the consumables database is accessible by the plurality of instruments such that a second selected instrument of the plurality of instruments is able to perform tests using the partially depleted consumable item based on the corresponding updated status and usage information stored in the consumables database.

## Claims

1. A system comprising:
a middleware system comprising a first interface and a second interface, wherein the middleware system is operatively coupled to or incorporated into a laboratory information system, LIS;
a consumables database;
machine-readable instructions; and
at least one processor circuit to execute the machine-readable instructions at the middleware system to:
access, via the first interface, instrument status information about a first instrument from the first instrument, the first instrument comprising a first intelligent instrument communicatively coupled to the first interface via a first communication protocol;
access, via the second interface, reagent status and usage information about a consumable item from the first instrument, the first instrument communicatively coupled to the second interface via a second communication protocol, the second interface different than the first interface, the second communication protocol comprising a custom protocol different than the first communication protocol, the consumable item removably installed in the first instrument, the first instrument to use the consumable item to form a partially depleted consumable item;
cause, via the first interface, the instrument status information to be stored in the LIS, the LIS being different than the consumables database; and
cause, via the second interface, the reagent status and usage information corresponding to the consumable item to be stored in the consumables database,
a second instrument to access the reagent status and usage information for the consumable item from the consumables database responsive to the partially depleted consumable item being removably installed in the second instrument.

2. The system of claim 1, wherein the instrument status information includes one or more of an operational state of the first instrument, a workload of the first instrument, quality control information generated by the first instrument for samples, or sample analysis results generated by the first instrument.

3. The system of claim 1, wherein the consumables information includes one or more of a number of tests conducted using the consumable item, a number of tests remaining for the consumable item, shelf-life expiration data for the consumable item, quality control information for the consumable item, or calibration information for the consumable item.

4. The system of claim 1, wherein the first communication protocol includes an HL7 communication protocol or an ASTM communication protocol and the second communication protocol is different than the HL7 communication protocol and the ASTM communication protocol.

5. The system of claim 4, wherein the second protocol is not compatible with the HL7 protocol or the ASTM protocol.

6. The system of claim 1, wherein the consumables information includes (a) first consumables information corresponding to a number of tests before depletion of the consumable item, the first consumables information associated with a first time, and (b) second consumables information corresponding to the number of tests before depletion of the consumable item, the second consumables information associated with a second time, the second time after the first time.

7. The system of claim 6, wherein one or more of the at least one processor circuit is to update the first consumables information in the consumables database based on the second consumables information.

8. The system of claim 1, wherein one or more of the at least one processor circuit is to update the consumables information in the consumables database responsive to an indication that the consumable item has been removed from the first instrument.

9. A method comprising:
accessing, via a first interface, instrument status information about a first instrument from the first instrument, the first instrument comprising a first intelligent instrument communicatively coupled to the first interface via a first communication protocol;
accessing, via a second interface, reagent status and usage information about a consumable item from the first instrument, the first instrument communicatively coupled to the second interface via a second communication protocol, the second interface different than the first interface, the second communication protocol comprising a custom protocol different than the first communication protocol, the consumable item removably installed in the first instrument, the first instrument to use the consumable item to form a partially depleted consumable item;
causing, via the first interface, the instrument status information to be stored in a laboratory information system, LIS; and
causing, via the second interface, the reagent status and usage information corresponding to the consumable item to be stored in a consumables database, the consumables database being different from the LIS,
accessing, by a second instrument, the reagent status and usage information for the consumable item from the consumables database responsive to the partially depleted consumable item being removably installed in the second instrument.

10. The method of claim 9, further including transmitting, by the second instrument via the second interface, instrument status information for the second instrument to the LIS.

11. The method of claim 9, further including transmitting, by the second instrument via the first interface and after the usage of the partially used consumable item by the second instrument, third consumable information to the consumables database for the partially used consumable item.

12. At least one memory comprising machine-readable instructions to cause at least one processor circuit to at least:
cause first consumables information for a reagent to be stored in a consumables database, the first consumables information received from a first instrument via a first interface, the first interface associated with a first communication protocol;
cause instrument status information for the first instrument to be stored in a laboratory information system, LIS, the instrument status information received from the first instrument via a second interface, the second interface associated with a second communication protocol, LIS database different than the consumables database, the second interface different than the first interface, the second communication protocol different than the first communication protocol; and
update the first consumables information for the reagent stored in the consumable database based on second consumables information for the reagent to generate third consumables information, the second consumables information received from a second instrument via the first interface, the second instrument different than the first instrument.

13. The at least one memory of claim 12, wherein the machine-readable instructions are to cause one or more of the at least one processor circuit to transmit the first consumables information to the second instrument via the first interface responsive to a request from the second instrument, the request indicative of a receipt of the reagent at the second instrument.

14. The at least one memory of claim 12, wherein the machine-readable instructions are to cause one or more of the at least one processor circuit to update the third consumables information based on fourth consumables information for the reagent received from the second instrument, the fourth consumables information received from the second instrument after the second consumables information.

15. The at least one memory of claim 12, wherein the first consumables information includes a first test count associated with the reagent and the second consumables information includes a second test count associated with the reagent.

16. The at least one memory of claim 12, wherein the instrument status information includes one or more of an operational state of the first instrument, a workload of the first instrument, or test results generated by the first instrument.
